# EUROPÄISCHE PATENTANMELDUNG

(11) **EP 1 347 326 A1**
(43) Veröffentlichungstag der Anmeldung: **24.09.2003**
(21) Anmeldenummer: 03006028.9
(22) Anmeldetag: 19.03.2003
(51) Int. Cl.: G02B 21/00, A61F 9/00

(54) **Operationsmikroskop mit Informationssystem**

(30) Priorität: 22.03.2002 DE 10212805
(71) Anmelder: Leica Microsystems (Schweiz) AG, 9435 Heerbrugg (CH)
(72) Erfinder: Sander, Ulrich, Dr., 9445 Rebstein (CH)
(74) Vertreter: Reichert, Werner F., Dr.

(57) **Zusammenfassung**

Die Erfindung betrifft ein Operationsmikroskop - beispielsweise ein Ophthalmo-(Stereo-)Operationsmikroskop - mit einem Informationssystem, das ein Dateneinspiegelungsmodul (5) und eine Elektronikeinheit (6) für das Ermitteln und Anzeigen von Patientendaten umfasst. Eine programmgesteuerte Verbindung (12) zwischen der Elektronikeinheit (6) und dem Dateneinspiegelungsmodul (5) übermittelt die angezeigten Parameter aus der Elektronikeinheit (6) an das Dateneinspiegelungsmodul (5), das diese Parameter ins Blickfeld eines Anwenders einspiegelt.

## Beschreibung

Die Erfindung betrifft ein (Stereo-)Operationsmikroskop mit einem Informationssystem, das ein Dateneinspiegelungsmodul und eine Elektronikeinheit für das Ermitteln und Anzeigen von Patientendaten - beispielsweise Bestrahlungsdaten des Patienten- , die im Mikroskopsystem gemessen werden, umfasst.

Es ist bekannt, dass bei Operationsmikroskopen, die in der Augenheilkunde angewendet werden, das Licht zur Beleuchtung des Patientenauges Netzhautschäden hervorrufen kann. Deshalb ist es sinnvoll, bestimmte Parameter der Beleuchtung zu messen, zu kontrollieren und zu registrieren. Diese Parameter sind beispielsweise die Lichtintensität der Beleuchtung, die Bestrahlungsdauer und die Bestrahlungsdosis.

Die Lichtintensität der Beleuchtung wird normalerweise sichtbar dargestellt; in der Regel über ein Anzeigemodul an der Steuerelektronik und/oder durch eine Markierung an einem Potentiometer zur Lichtintensitätseinstellung. Die Bestrahlungsdauer wird mittels einer Zeiterfassung (Uhr) gemessen und die Bestrahlungsdosis wird durch den Operateur aus den beiden Parametern Lichtintensität und Bestrahlungsdauer ermittelt.

Eine Dosismessung in Verbindung mit einem Operationsmikroskop wird in der US-A-4 657 013 und der US-A-4 682 595 offenbart.

Die Bestrahlungszeit und die Bestrahlungsdosis könnten auch auf einem eigenen Gerät angezeigt oder auch an die Steuerelektronik des Mikroskops weitergeleitet und dort in einfacher Weise angezeigt werden.

Aus Firmen-Publikationen der Leica Microsystems AG ist es bereits bekannt, Einspiegelungen in das Betrachtungsfeld des Chirurgen für verschiedene Zwecke - insbesondere für Video-, Endoskop- oder Patientendaten-Einblendungen - mit dem sogenannten Imaging-Modul ('The Leica Imaging Module', Publikations-Datum: Oktober 1999) oder dessen Nachfolgemodell DI-Modul ('The Leica Dual Imaging and the Ultra Observer', Publikations-Jahr 2001) technisch zu realisieren. Als weiterer Stand der Technik wird auf die WO 01/27659 A2 und die WO 01/48528 A2 sowie auf die nicht vorveröffentlichte DE-Patentanmeldung mit dem amtlichen Aktenzeichen 101 57 613.7 hingewiesen.

Diese Module werden am Operationsmikroskop zwischen dem Zoomsystem und dem Beobachtertubus angesetzt. Die darzustellenden Parameter werden beispielsweise von einer Mess- oder Endoskopeinheit zum Imaging-Modul gesendet, und zwar per elektrischem Verbindungskabel oder per Lichtleiter - vgl. hierzu die EP 102 901.6 - oder per Funk- vgl. hierzu die nicht vorveröffentlichte DE-Patentanmeldung mit dem amtlichen Aktenzeichen 102 02 125.2. Die im Display dieser Module angezeigten Video- oder Patientendaten werden damit im Betrachtungsfeld des Chirurgen angezeigt.

Eine andere Variante besteht darin, die Daten statt in die Zwischenbildebene zwischen Zoomsystem und Beobachtertubus direkt ins Okular einzuspiegeln.

Der Erfinder erkannte, dass diese bekannten Systeme unter anderem folgende Nachteile aufweisen:

Da sich die Steuerelektronik des Mikroskops weitab vom Betrachtungsfeld des Chirurgen befindet und der Chirurg nicht dauernd die Anzeigen im Auge behalten kann, ist der Standort der Anzeigen in der Anwendung unpraktisch: Es muss weiteres Personal die Anzeigen überwachen und verbal Rückmeldung an den Chirurgen machen.

Durch den wechselnden Blickkontakt des Chirurgen zwischen den Anzeigemodulen und dem Operationsfeld entstehen Akkommodationsprobleme für das Auge des Chirurgen, die jedenfalls Zeit verbrauchen.

Nicht alle oben erwähnten Daten werden bisher direkt an der Steuerelektronik des Mikroskops angezeigt, sondern teilweise auf separaten Geräten.

Durch akustische Signale kann der Chirurg zwar auf sicherheitsrelevante Daten aufmerksam gemacht werden, muss dann jedoch trotzdem seinen Blick vom Operationsfeld nehmen, um den aktuellen Status - wenigstens kontrollhalber - zu registrieren und zu interpretieren.

Die laufende Überwachung, wieviel der zulässigen Bestrahlungsdosis bereits vergeben wurde, ist ebenso nur durch ein Abweichen des Blickes des Chirurgen vom Operationsfeld hin zur Anzeige des Dosismessgerätes oder durch Einschalten von weiterem (Hilfs-) Personal möglich.

Aufgabe der Erfindung ist es demzufolge, ein Informationssystem für ein Operationsmikroskop zu schaffen, das die obigen Mängel beseitigt und daher ein sicheres und schnelleres Arbeiten für den Chirurgen möglich ist.

Gelöst wird diese Aufgabe durch das nachfolgend beschriebene Mikroskop mit einem Informationssystem:

Die auf einer Elektronikeinheit angezeigten Parameter, beispielsweise für Lichtintensität, Bestrahlungsdauer und/oder Bestrahlungsdosis, werden mittels einer Elektronikeinheit aufbereitet und erfindungsgemäß an ein an sich bekanntes Dateneinspiegelungsmodul übertragen. Dieses spiegelt die Parameter ins Blickfeld des Chirurgen. Die Übertragung von der Elektronikeinheit zum Dateneinspiegelungsmodul kann konventionell elektrisch, aber auch mittels Glasfaser oder Funksystemen erfolgen, wobei Funksysteme elektromagnetische Wellen, beispielsweise Infrarot- oder Radiowellen, sowie Schallwellen umfassen.

Zusätzlich lassen sich erfindungsgemäß auch Schwellen- oder Grenzwerte ins Blickfeld des Chirurgen einspiegeln.

Das Dateneinspiegelungsmodul spiegelt somit erfindungsgemäß alle für die Bestrahlung des Auges relevanten Parameter dauernd oder wahlweise ins Betrachtungsfeld des Chirurgen ein.

Die Anzeige der Daten kann gemäß einer Weiterentwicklung auch so geregelt werden, dass im ausgeblendeten Zustand sicherheitsrelevanter Daten das System diese Daten automatisch in das Beobachtungsfeld des Chirurgen weitergibt, sollte ein Schwellenwert erreicht oder sogar überschritten werden. Zum Beispiel blinkt erfindungsgemäß ein Symbol 'Uhr', wenn die Bestrahlungszeit überschritten wird.

Durch das vorgängig beschriebene neue Informationssystem in einem Operationsmikroskop werden unter anderem die folgenden Verbesserungen erreicht:

Es ergeben sich keine Einschränkungen der chirurgischen Tätigkeit durch ein Abwenden des Blickes des Chirurgen vom Operationsfeld.

Keine Akkommodationsprobleme für das Auge des Chirurgen.

Sicherheitsrelevante Parameter können bei Bedarf dauernd und vom Chirurgen selbst überwacht werden.

Der Chirurg kann seine Operations-Technik zeitlich besser planen und damit das Patientenauge besser schützen, da er die Bestrahlungsparameter während der Operation jederzeit einsehen kann.

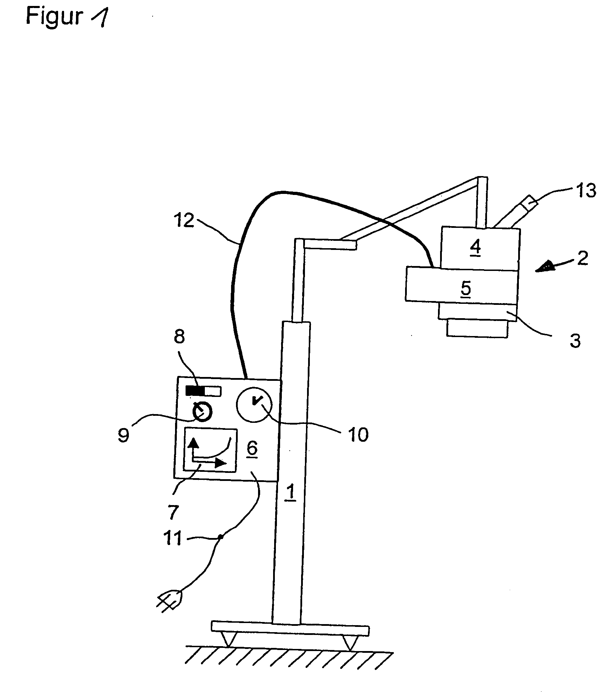

In der Zeichnung wird ein erfindungsgemäßes Operationsmikroskop mit einem Informationssystem schematisch dargestellt:

Dargestellt ist ein an einem Stativ 1 befestigtes Mikroskop 2 mit einem Zoom 3, einem Tubus 4, einem Okular 13, einem Dateneinspiegelungsmodul 5 und einer Elektronikeinheit 6. Die Elektronikeinheit 6 umfasst beispielsweise eine Dosisanzeige 7, eine Anzeige der Lichtintensität 8, ein Potentiometer 9 zur Lichtintensitätseinstellung, eine Zeiterfassungseinheit 10 sowie eine Netzverbindung 11.

Die an der Elektronikeinheit 6 dargestellten Parameter werden in dieser aufbereitet und mittels einer Verbindung 12 zur Datenübertragung an das Dateneinspiegelungsmodul 5 weitergeleitet und mit dem Dateneinspiegelungsmodul 5 bei Bedarf in das Blickfeld des Chirurgen eingespiegelt.

Gemäß einer Weiterentwicklung werden die gewünschten Werte, Handlungen und/oder Parameter - wie beispielsweise Einspiegeln, Ausblenden, Abschalten, Auto-Modus - mittels einer Blicksteuerung, beispielsweise mit der in der EP 788 613 B1 beschriebenen Vorrichtung, oder mittels einer Sprachsteuerung durch den Chirurgen angewählt.

### Bezugszeichenliste

- 1: Stativ
- 2: Mikroskop
- 3: Zoom
- 4: Tubus
- 5: Dateneinspiegelungsmodul
- 6: Elektronikeinheit
- 7: Dosisanzeige
- 8: Anzeige der Lichtintensität
- 9: Potentiometer zur Lichtintensitätseinstellung
- 10: Zeiterfassungseinheit
- 11: Netzverbindung
- 12: Verbindung zur Datenübertragung
- 13: Okular

## Patentansprüche

1. Operationsmikroskop, beispielsweise ein Ophthalmo(Stereo-)Operationsmikroskop, mit einem Informationssystem, das ein Dateneinspiegelungsmodul (5) und eine Elektronikeinheit (6) für das Ermitteln und Anzeigen von Patientendaten umfasst, **dadurch gekennzeichnet, dass** eine programmgesteuerte Verbindung (12) zwischen der Elektronikeinheit (6) und dem Dateneinspiegelungsmodul (5) vorgesehen ist, die im Betriebszustand die angezeigten Parameter aus der Elektronikeinheit (6) an das Dateneinspiegelungsmodul (5) übermittelt und das Dateneinspiegelungsmodul (5) diese Parameter ins Blickfeld eines Anwenders einspiegelt.

2. Operationsmikroskop nach Anspruch 1, **dadurch gekennzeichnet, dass** zu den dargestellten Parametern des Patienten die Lichtintensität der Bestrahlung, die Bestrahlungsdauer und/oder die Bestrahlungsdosis gehören.

3. Operationsmikroskop nach einem der vorhergehenden Ansprüche, **dadurch gekennzeichnet, dass** die Elektronikeinheit (6) so ausgebildet ist, dass zusätzlich zu den Parametern der Elektronikeinheit (6) Vergleichs- und/oder Schwellenwerte in das Blickfeld des Anwenders einspiegelbar sind.

4. Operationsmikroskop nach einem der vorhergehenden Ansprüche, **dadurch gekennzeichnet, dass** für die Übertragung der einzuspiegelnden Parameter von der Elektronikeinheit (6) zum Dateneinspiegelungsmodul (5) am Mikroskop (2) Übertragungskabel, Lichtleiter oder Funksysteme vorgesehen sind.

5. Operationsmikroskop nach einem der vorhergehenden Ansprüche, **dadurch gekennzeichnet, dass** die aktuellen Bestrahlungsparameter wahlweise kontinuierlich und/oder diskontinuierlich einspiegelbar sind.

6. Operationsmikroskop nach einem der vorhergehenden Ansprüche, **dadurch gekennzeichnet, dass** an der Elektronikeinheit (6) Schwellenwerte für die Beleuchtungsintensität und/oder die Bestrahlungsdauer und/oder die Bestrahlungsdosis setzbar sind.

7. Operationsmikroskop nach einem der vorhergehenden Ansprüche, **dadurch gekennzeichnet, dass** das Programm der Elektronikeinheit (6) so ausgebildet ist, dass sich die Anzeige beim Überschreiten der Schwellenwerte automatisch einschaltet und visuelle und/oder akustische Warnsignale abgibt.

8. Operationsmikroskop nach einem der vorhergehenden Ansprüche, **dadurch gekennzeichnet, dass** eine Blicksteuerung vorgesehen ist, die die gewünschten Werte und Parameter - beispielsweise Einspiegeln, Ausblenden, Abschalten und Auto-Modus - mittels Augenbewegung des Anwenders anwählt.

9. Operationsmikroskop nach einem der vorhergehenden Ansprüche, **dadurch gekennzeichnet, dass** eine Sprachsteuerung vorgesehen ist, die die gewünschten Werte und Parameter - beispielsweise Einspiegeln, Ausblenden, Abschalten und Auto-Modus - mittels einer Spracherkennung des Anwenders anwählt.
